(19) Europäisches Patentamt
European Patent Office
Office européen des brevets

(11) Numéro de publication: **0 164 452**
**A1**

# (12) DEMANDE DE BREVET EUROPEEN

(21) Numéro de dépôt: **84200843.5**

(22) Date de dépôt: **13.06.84**

(51) Int. Cl.[4]: **C 07 D 231/24**
//C07D237/22, C07D239/69,
A61K31/635

(43) Date de publication de la demande:
**18.12.85 Bulletin 85/51**

(84) Etats contractants désignés:
**BE DE FR GB IT LU NL**

(71) Demandeur: **Massague Vendrell, Antonio Javier**
**C/Margenat No. 1**
**Barcelone(ES)**

(71) Demandeur: **Massague Vendrell, Juan**
**San Antonio Maria Claret No. 51**
**Barcelone 25(ES)**

(71) Demandeur: **LIEBHERR S.A.**
**21, rue de Veyrot**
**CH-1217 Meyrin/Genève(CH)**

(72) Inventeur: **Massague Vendrell, Antonio Javier**
**c/ Margenat No. 1**
**Barcelone(ES)**

(72) Inventeur: **Massague Vendrell, Juan**
**San Antonio Maria Claret No. 51**
**Barcelone 25(ES)**

(74) Mandataire: **Hranitzky, Wilhelm Max et al,**
**c/o WILLIAM BLANC & CIE Conseils en Propriété Industrielle SA 5, Place du Molard**
**CH-1204 Genève(CH)**

(54) **Combinaison hydrosoluble de diméthyl-1,5-phényl-2-pyrazol-3-one et de sulfamide et procédé pour sa préparation.**

(57) Cette combinaison hydrosoluble de diméthyl-1,5-phényl-2-pyrazol-3-one (composé connu, par exemple, sous le nom de "Phénazone") et de sulfamide est constituée par un composé salin de formule générale:

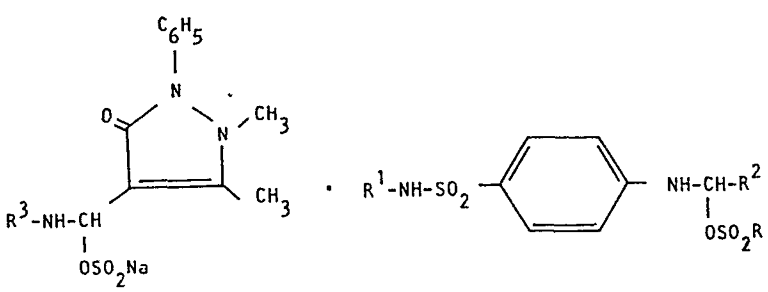

(I)

dans laquelle R est un atome de métal alcalin et R[1] et R[2] représentent chacun un atome d'hydrogène ou un reste aliphatique, aromatique, hétérocyclique ou un groupement d'au moins deux de ces restes. Elle permet d'obtenir simultanément les effets thérapeutiques de la phénazone et d'une sulfamide, tout en se prêtant à la préparation d'une solution aqueuse stable renfermant le principe actif sous une forme facilement hydrolysable dans l'organisme.

EP 0 164 452 A1

Croydon Printing Company Ltd

COMBINAISON HYDROSOLUBLE DE DIMETHYL-1,5-PHENYL-2-PYRAZOL-3-ONE ET DE SULFAMIDE ET PROCEDE POUR SA PREPARATION

La présente invention a pour objet une combinaison hydrosoluble de diméthyl-1,5-phényl-2-pyrazol-3-one (composé connu, par exemple, sous le nom de "Phénazone" ou d'"Antipyrine") et de sulfamide ainsi qu'un procédé de fabrication de cette combinaison.

L'utilisation des sulfamides, sous forme de sels sodiques, en médecine humaine et vétérinaire, est considérablement gênée par le fait que la forte alcalinité de tels sels les rend impropres à l'emploi dans des médicaments destinés à être administrés par voie orale ou parentérale. En effet, les solutions aqueuses de ces sels sont instables et s'altèrent au simple contact de l'air ainsi qu'en présence des sels contenus dans l'eau de boisson. En outre, en cas d'administration par voie parentérale, ces sels sont susceptibles de provoquer une forme irritation des tissus qui peut aller jusqu'à la dégénérescence et la nécrose de ces derniers.

D'autre part, on connaît des composés de formule générale :

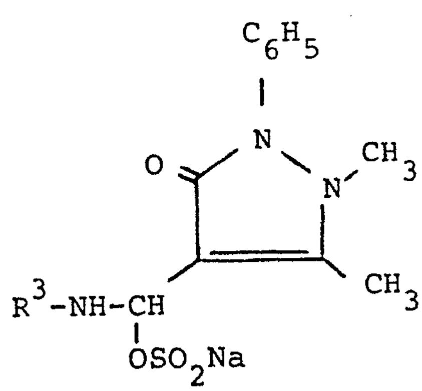

(V)

dans laquelle $R^3$ est un reste provenant d'une molécule de sulfamide, par exemple le reste ayant la formule

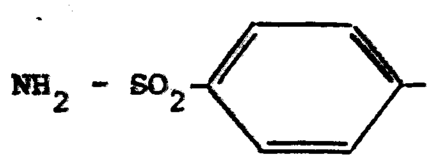

(VI)

ainsi que des composés de formule générale

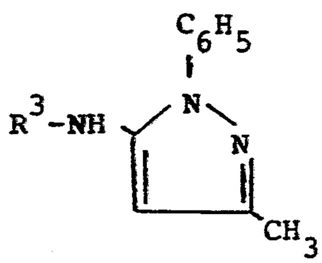

(VII)

dans laquelle $R^3$ a la même signification que dans la formule (IV) indiquée ci-dessus. Ces composés présentent l'avantage de permettre de combiner en un seul médicament les effets antibactériens des sulfamides et les effets analgésiques et antipyrétiques de la phénazone. Les composés de formule (V) sont décrits dans le brevet des Etats-Unis d'Amérique No 2 650 219 et les composés de formule (VII), qui sont commercialisés sous le nom de sulfaméthylphénazole sont décrits, par exemple, dans la publication suivante : Crippa, Garneri, Gazz. Chim. Ital. 85, 199 (1955) et font l'objet du brevet britannique No 848 627.

L'invention a pour but de fournir une combinaison hydrosoluble de "phénazone" et de sulfamides, permettant d'obtenir simultanément les effets thérapeutiques de la phénazone et d'une sulfamide, cette combinaison étant sous une forme qui se prête à la préparation d'une solution aqueuse stable renfermant le principe actif sous une forme facilement hydrolysable dans l'organisme afin de faciliter, respectivement, son administration et

son assimilation par l'organisme.

A cet effet, la combinaison selon l'invention est caractérisée en ce qu'elle est constituée par un composé salin de formule générale :

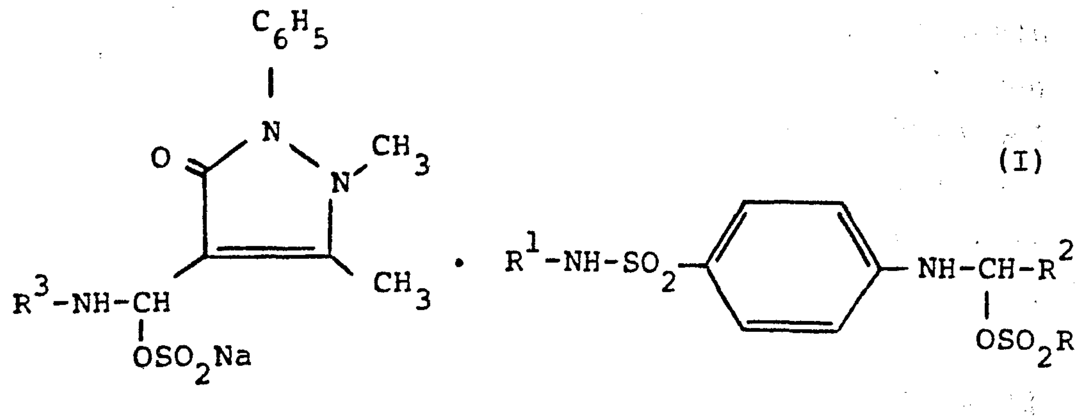

(I)

dans laquelle R est un atome de métal alcalin et $R^1$ et $R^2$ représentent chacun un atome d'hydrogène ou un reste aliphatique,aromatique, hétérocyclique ou un groupement d'au moins deux de ces restes.

De préférence, R est le sodium tandis que le reste $R^1$ est l'hydrogène ou un groupe choisi parmi les suivants : 4,6-diméthyl-2-pyrimidyl; 6-méthoxy-3-pyridazinyl et 2-thiazolyl le reste $R_2$ étant l'hydrogène ou l'un des groupes suivants : méthyl; éthyl et phényl-éthylène.

Il est à remarquer que la combinaison selon l'invention se présente sous forme d'une poudre blanche ou jaunâtre, facile à manipuler et à conserver, alors que les simples mélanges de phénazone avec les dérivés obtenus par la réaction de composés aldéhydo-bisulfitiques avec les sulfamides sont sous forme de conglomérats caoutchouteux qui sont impropres à l'emploi en tant que substance active de médicaments. D'autre part, les solutions aqueuses de cette combinaison sont complètement transparentes et restent stables pendant une période pratiquement illimitée, ce qui facilite leur administration aussi

bien par voie orale que par voie parentérale. Au contraire, les solutions aqueuses des composés obtenus par réaction des composés aldéhydo-bisulfitiques avec les sulfamides correspondantes, par exemple le dérivé disulfitique de sulfaméthoxypyridazine méthane(obtenu par réaction de dérivés bisulfitiques sodiques de formaldéhyde avec la sulfaméthoxypyridazine (4-amino-N-(6-méthoxy-3-pyridazinyl) benzène sulfonamide)) précipitent spontanément au bout de quelques heures après leur préparation.

Le procédé de fabrication de la combinaison selon l'invention présente les caractéristiques spécifiées dans la revendication 2.

Ce procédé consiste donc à provoquer la salification de la phénazone par un dérivé aldéhydo-bisulfitique alcalin de sulfamide. La préparation de ces dérivés aldéhydo-bisulfitiques alcalins de sulfamide est effectuée de manière connue en soi, par exemple en faisant réagir, en milieu aqueux, un dérivé bisulfitique alcalin d'un aldéhyde , par exemple le composé bisulfitique sodique du formaldéhyde ou de l'acétaldéhyde sur la sulfamide correspondante.

Exemple 1

Combinaison hydrosoluble de "phénazone" et de "sulfaméthoxypyridazine" (4-amino-N-(6-méthoxy-3-pyridazine) benzène sulfonamide)

On dissout 28 g de sulfaméthoxypyridazine et 13,4 g de dérivé bisulfitique sodique de formaldéhyde dans 100 ml d'eau puis on chauffe, à 60°C, pendant 15 minutes, la solution ainsi obtenue et on ajoute ensuite lentement,

au milieu réactionnel, 18,8 g de "phénazone" en solution dans 50 ml d'eau.

On laisse réagir pendant 30 minutes à 60°C, tout en agitant puis on décolore le milieu réactionnel au moyen de charbon actif et on filtre à chaud. Après quoi,on concentre le filtrat sous vide, en veillant à ce que la température ne dépasse pas 60°C, pendant cette opération. On obtient 56,5 g de produit final, sous forme d'une poudre blanche, soluble dans l'eau à la température ambiante.

Exemple 2

Combinaison hydrosoluble de "phénazone" et de "sulfaméthazine" (4-amino-N-(4,6-diméthyl-2-pyrimidinyl)benzène sulfonamide.

On dissout 27,8 g de sulfaméthazine et 15,7 g de dérivé bisulfitique sodique d'acétaldéhyde dans 50 ml d'eau puis on chauffe à 50°C pendant 30 minutes. Après quoi, on décolore le milieu réactionnel avec du charbon actif et on le filtre à chaud. On refroidit, à 10°C, la solution ainsi obtenue et on précipite le produit de la réaction par addition d'alcool éthylique. On filtre le précipité et on le lave à l'éthanol. On obtient ainsi 38,7 g de dérivé bisulfitique sodique de sulfaméthazine-éthane.

On dissout ensuite la totalité de ce dernier dérivé dans 50 ml d'eau à 60°C et on ajoute lentement à la solution ainsi obtenue 18,40 g de "phénazone" en solution dans 50 ml d'eau.

On laisse réagir pendant 30 minutes, en maintenant la température à 60°C, tout en agitant, puis on décolore le

milieu réactionnel avec du charbon actif et on le filtre à chaud . On lyophilise le filtrat ce qui permet d'obtenir 56 g de produit final sous forme d'une poudre blanche.

## REVENDICATIONS

1. Combinaison hydrosoluble de diméthyl-1,5-phényl-2-pyrazol-3-one et de sulfamide, caractérisée en ce qu'elle est constituée par un composé salin de formule générale :

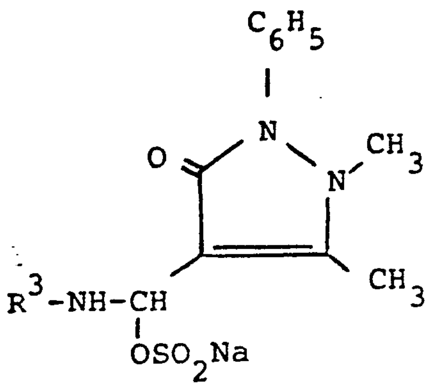

•

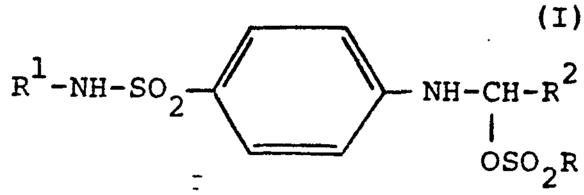

(I)

dans laquelle R est un atome de métal alcalin et $R^1$ et $R^2$ représentent chacun un atome d'hydrogène ou un reste aliphatique, aromatique, hétérocyclique ou un groupement d'au moins deux de ces restes.

2. Procédé de fabrication d'une combinaison hydrosoluble de diméthyl-1,5-phényl-2-pyrazol-3-one et de sulfamide, répondant à la formule (I), spécifiée dans la revendication 1, caractérisé par le fait que l'on fait réagir un dérivé bisulfitique alcalin d'aldéhyde, ayant la formule :

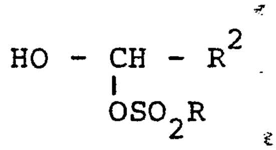

(II)

avec une sulfamide de formule générale :

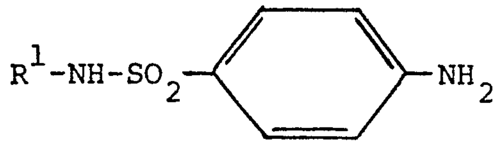

(III)

R, $R^1$ et $R^2$ ayant les significations indiquées dans la revendication 1, de façon à former un dérivé aldéhydo-bisulfitique alcalin de sulfamide, ayant la formule générale :

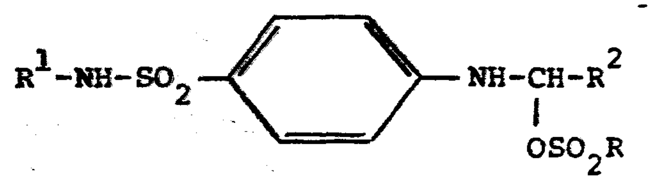

(IV)

et que l'on provoque la salification de la diméthyl-1,5-phényl-2-pyrazol-3-one par le dérivé de formule (IV) ainsi obtenu, en maintenant la température de réaction à une valeur inférieure aux températures de polymérisation des composés de départ, de façon à former le composé salin répondant à la formule (I).

Office européen des brevets

# RAPPORT DE RECHERCHE EUROPEENNE

0164452
Numéro de la demande

EP 84 20 0843

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,A | US-A-2 650 219 (J.H.T. LEDRUT) * Revendication; colonne 7, lignes 45-62 * | 1,2 | C 07 D 231/24 // C 07 D 237/22 C 07 D 239/69 A 61 K 31/635 |
| A | GB-A-1 035 463 (S.P.A. ISTITUTO GENTILI) * Revendications 1-3 * | 1,2 | |
| A | US-A-2 712 012 (J.H. CLARK) * Revendications 1-3 * | 2 | |
| A | DD-A- 844 (W. HENTRICH et al.) * Exemples 1,2 * | 2 | |
| A | DE-C- 229 814 (A. VOSWINKEL) * Revendication * | 2 | DOMAINES TECHNIQUES RECHERCHES (Int. Cl.4) |
| | | | A 61 K 31/00 C 07 C 143/00 C 07 D 231/00 C 07 D 237/00 C 07 D 239/00 |

Le présent rapport de recherche a été établi pour toutes les revendications

| Lieu de la recherche | Date d'achèvement de la recherche | Examinateur |
|---|---|---|
| BERLIN | 31-01-1985 | HASS C V F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82